(19) Europäisches Patentamt
European Patent Office
Office européen des brevets

(11) Veröffentlichungsnummer: **0 069 926**
**B1**

(12) # EUROPÄISCHE PATENTSCHRIFT

(45) Veröffentlichungstag der Patentschrift: **25.09.85**

(51) Int. Cl.⁴: **C 07 C 69/60**, C 07 C 67/08

(21) Anmeldenummer: **82105868.2**

(22) Anmeldetag: **01.07.82**

(54) **Verfahren zur Herstellung von Fumarsäuremonoestern.**

(30) Priorität: **11.07.81 DE 3127432**

(43) Veröffentlichungstag der Anmeldung: **19.01.83 Patentblatt 83/3**

(45) Bekanntmachung des Hinweises auf die Patenterteilung: **25.09.85 Patentblatt 85/39**

(84) Benannte Vertragsstaaten: **BE CH DE FR GB IT LI NL**

(56) Entgegenhaltungen:
**DE - A - 1 165 586**
**FR - A - 1 512 243**
**GB - A - 1 089 809**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Zecher, Wilfried, Dr., Treptower Strasse 6, D-5090 Leverkusen 1 (DE)**
Erfinder: **Merten, Rudolf, Dr., Berta-von-Suttner-Strasse 55, D-5090 Leverkusen 1 (DE)**

**EP 0 069 926 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein Verfahren zur Herstellung von Fumarsäuremonoestern aus Maleinsäureanhydrid.

Es ist bekannt, dass man Fumarsäuremonoalkylester durch partielle Veresterung der Fumarsäure mit Alkoholen erhält [Chem. Ber. 30, 2651 (1897)]. Weitere bekannte Herstellungswege sind die partielle Hydrolyse von Fumarsäurediestern und die cis-trans-Umlagerung von Maleinsäuremonoestern (DE-PS 1 291 739 und DE-OS 2 130 300). Die Umlagerung von Maleinsäuremonoester in Gegenwart von cis-trans-Katalysatoren wie Iod wird in der FR 1 512 243 beschrieben.

In der GB 1 089 809 wird die Umsetzung von Maleinsäureanhydrid mit Hydroxyverbindungen ohne cis-trans-Katalysator beschrieben. Bei diesem Verfahren erhält man Fumarsäuremonoester nur mit geringen Ausbeuten.

Aus der DE-AS 1 165 586 ist die zweistufige Herstellung von Fumarsäuremonoalkylester bekannt. In einer ersten Stufe werden die Maleinsäurehalbester durch Umsetzung von Maleinsäureanhydrid mit Alkoholen hergestellt. In einer zweiten Stufe werden danach die Maleinsäurehalbester in Gegenwart einer geringen Menge eines Carbonsäurechlorids als Isomerisierungskatalysator umgesetzt.

Es wurde ein Verfahren zur Herstellung von Fumarsäuremonoestern gefunden, das dadurch gekennzeichnet ist, dass man in eine Lösung oder Schmelze aus gegebenenfalls substituiertem Maleinsäureanhydrid in Gegenwart eines cis-trans-Katalysators eine Hydroxyverbindung im Temperaturbereich von 100 bis 220°C der Umsetzung entsprechend einträgt.

Das erfindungsgemässe Verfahren ist überraschend, da unter den erfindungsgemässen Reaktionsbedingungen die Rückspaltung der Halbester und weiterhin die Bildung eines erheblichen Anteils an Diestern zu erwarten ist.

Im Vergleich zu bekannten Verfahren werden erfindungsgemäss gute Ausbeuten und sehr kurze Reaktionszeiten erzielt. Besonders vorteilhaft ist, dass man bei dem Einsatz flüchtiger Alkohole das Arbeiten unter Druck vermeiden kann.

Das erfindungsgemässe Verfahren kann beispielsweise durch das folgende Reaktionsschema erläutert werden:

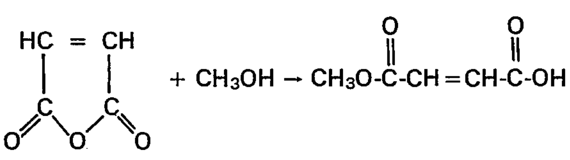

Das Maleinsäureanhydrid für das erfindungsgemässe Verfahren kann durch Alkyl, Aryl oder Halogen substituiert sein.

Alkyl ist hierbei bevorzugt Niederalkyl ($C_1$ bis etwa $C_6$), insbesondere bevorzugt Methyl und Ethyl.

Aryl ist hierbei bevorzugt Phenyl, Tolyl und Xylyl.

Halogen ist hierbei bevorzugt Fluor, Chlor, Brom oder Jod, insbesondere bevorzugt Chlor.

Für das erfindungsgemässe Verfahren sind Maleinsäureanhydride der Formel (I)

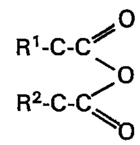

(I)

in der

$R^1$ und $R^2$ gleich oder verschieden sind und Wasserstoff, Niederalkyl, Aryl oder Halogen bedeuten,

bevorzugt.

Beispielsweise seien die folgenden Maleinsäureanhydride genannt: Maleinsäureanhydrid, Dimethylmaleinsäureanhydrid, Monoethylmaleinsäureanhydrid, Monophenylmaleinsäureanhydrid, Dichlormaleinsäureanhydrid und Monobrommaleinsäureanhydrid.

Im besonderen wird unsubstituiertes Maleinsäureanhydrid für das erfindungsgemässen Verfahren bevorzugt.

Hydroxyverbindungen für das erfindungsgemässe Verfahren können Verbindungen der Formel (II)

$$R^3(OH)_n \quad (II)$$

in der

$R^3$ Alkyl, Alkenyl, Alkinyl, Aralkyl, Aryl, Cycloalkyl, einen 5- oder 6gliedrigen heterocyclischen Rest mit 1 oder 2 Sauerstoff- oder Stickstoffatomen, oder den Rest eines Polyethers, Polyesters, Polyharnstoffs, Polyurethans, Polyimids oder Polyhydantoins bedeutet und

n für eine der Zahlen 1, 2 oder 3 steht,

sein.

Alkyl kann erfindungsgemäss hierbei ein geradkettiger oder verzweigter Kohlenwasserstoff mit 1 bis 24 Kohlenstoffatomen, bevorzugt 1 bis 12 Kohlenstoffatomen, sein. Insbesondere bevorzugt wird der Niederalkylrest mit 1 bis etwa 6 Kohlenstoffatomen. Beispielsweise seien die folgenden Alkylreste genannt: Methyl, Ethyl, Propyl, Isopropyl, Butyl, Isobutyl, Pentyl, Isopentyl, Hexyl- und Isohexyl.

Alkenyl kann erfindungsgemäss hierbei ein geradkettiger oder verzweigter Kohlenwasserstoff mit einer oder mehreren, bevorzugt einer, Doppelbindung sein, der 3 bis 24, bevorzugt 3 bis 12, Kohlenstoffatome enthält. Insbesondere bevorzugt wird ein Niederalkenylrest mit 3 bis etwa 6 Kohlenstoffatomen. Beispielsweise seien die folgenden Alkenylreste genannt: 2-Propenyl-, Isobutenyl-, 2-Methyl-3-butenyl- und 9-Octadecenyl.

Alkinyl kann erfindungsgemäss hierbei ein geradkettiger oder verzweigter Kohlenwasserstoff mit einer oder mehreren, bevorzugt einer, Dreifachbindung sein und 3 bis 24, bevorzugt 3 bis 12, Kohlenstoffatome enthalten. Insbesondere bevorzugt ist ein Niederalkinylrest mit 3 bis etwa 6 Kohlenstoffatomen. Beispielsweise seien die folgenden Alkinylreste genannt: 2-Propinyl-, 3-Butin-2-yl-, und 2-Methyl-3-butinyl und 9-Octadecenyl.

Aralkyl kann erfindungsgemäss hierbei 1 bis 6, bevorzugt 1 oder 2, Kohlenstoffatome im aliphatischen Teil und 6 bis 12, bevorzugt 6 bis 10 Kohlenstoffatome im aromatischen Teil enthalten. Beispielsweise

seien die folgenden Aralkylreste genannt: Benzyl, Phenylethyl- und Phenylpropyl.

Aryl kann erfindungsgemäss hierbei 6 bis 12 Kohlenstoffatome enthalten. Bevorzugte Arylreste sind Phenyl, Biphenyl und Naphthyl. Insbesondere bevorzugt ist der Phenylrest.

Cycloalkyl kann erfindungsgemäss hierbei ein cyclischer aliphatischer Kohlenwasserstoff mit 2 bis 10, bevorzugt 5 bis 7, Kohlenstoffatomen sein. Insbesondere bevorzugt wird Cyclopentyl und Cyclohexyl. Beispielsweise seien die folgenden Cycloalkylreste genannt: Cyclopropyl, Cyclobutyl, Cyclopentyl, Cyclohexyl, Cycloheptyl, Cyclooctyl, Cyclononyl und Cyclodecyl.

Ein 5- oder 6gliedriger heterocyclischer Rest mit 1 oder 2 Sauerstoffatomen kann vorzugsweise ein Furan- oder Dioxanring sein, der gegebenenfalls über Alkylen ($C_1$ bis $C_6$) mit der Hydroxygruppe verbunden ist.

Ein Polyetherrest kann erfindungsgemäss hierbei aus den Manomeren Ethylenoxid oder Propylenoxid aufgebaut sein. Die Polyetherreste haben erfindungsgemäss ein Molekulargewicht im Bereich von 75 bis 20 000, bevorzugt von 500 bis 5000.

Ein Polyesterrest kann erfindungsgemäss hierbei aus den monomeren Bausteinen Phthalsäure, Isophthalsäure, Terephthalsäure, Adipinsäure und Ethylenglykol, Neopentylglykol, Trimethylolpropan und Glycerin aufgebaut sein. Die erfindungsgemässen Polyesterreste haben im allgemeinen ein Molekulargewicht im Bereich von 200 bis 30 000, bevorzugt von 300 bis 10 000.

In ähnlicher Weise kann der $R^3$ auch für einen Polyharnstoffrest, einen Polyurethanrest, einen Polyimidrest oder einen Polyhydantoinrest stehen. Diese Reste haben im allgemeinen ein Molekulargewicht im Bereich von 500 bis 30 000.

Die genannten Reste $R^1$ können selbstverständlich durch alle Reste substituiert sein, die sich unter den erfindungsgemässen Bedingungen nicht verändern. Beispielweise seien die folgenden Reste genannt: Halogen, Nitro, Alkyl, Alkoxy, Aroxy, Alkylmercapto, Arylmercapto, Sulfonsäuren und Carbonsäuren und deren Ester.

Bevorzugte Hydroxyverbindungen für das erfindungsgemässe Verfahren sind Verbindungen der Formel (III)

$$R^4(OH)_n \qquad (III)$$

in der

$R^4$ Alkyl mit 1 bis 24 Kohlenstoffatomen, Alkenyl mit 3 bis 24 Kohlenstoffatomen, Alkinyl mit 3 bis 24 Kohlenstoffatomen, Aralkyl mit 1 bis 6 Kohlenstoffatomen im aliphatischen Teil und 6 bis 12 Kohlenstoffatomen im aromatischen Teil, Aryl mit 6 bis 12 Kohlenstoffatomen oder Cycloalkyl mit 5 bis 10 Kohlenstoffatomen bedeutet und

n die obengenannte Bedeutung hat.

Beispielsweise seien die folgenden Hydroxyverbindungen genannt: Methanol, Ethanol, n-Butanol, iso-Butanol, tert.-Butanol, Hexanol, Eicosanol, Allylalkohol, Propargylalkohol, Cyclohexanol, Phenol und Benzylalkohol.

Für das erfindungsgemässe Verfahren werden aliphatische Hydroxyverbindungen wie Methanol, Ethanol, Isopropanol, Allylalkohol und Propargylalkohol besonders bevorzugt.

Das erfindungsgemässe Verfahren wird in Gegenwart eines cis-trans-Katalysators durchgeführt. Dies sind Katalysatoren, die eine cis-trans-Umlagerung beschleunigen [Theoretische Grundlagen der organischen Chemie, 2. Auflage, 1. Band, Seite 477 (1956)]. Beisielsweise seien die folgenden cis-trans-Katalysatoren genannt: Halogene, wie Fluor, Chlor, Brom und Jod; Säuren, z.B. Mineralsäuren wie Halogenwasserstoffsäuren und organische Säuren, wie z.B. p-Toluolsulfonsäure, Säurehalogenide, wie Acetylchlorid und Benzolsulfochlorid, Titantetrachlorid, Bortrifluorid, Metalle wie Platin, die Kationen der Alkalimetalle wie das Natriumion, Amine wie Triethylamin, Morpholin, Piperidin, Anilin, Ammoniumsalze wie Ammoniumchlorid, Thioharnstoff und Phosphorverbindungen wie Triethylphosphit und Triphenylphosphin. Für das erfindungsgemässe Verfahren wird Jod als cis-trans-Katalysator besonders bevorzugt.

Das erfindungsgemässe Verfahren wird in einer Lösung oder einer Schmelze der Reaktanten durchgeführt. Es können übliche Lösungsmittel verwendet werden, die unter den Reaktionsbedingungen nicht reagieren oder nur lockere Additionsverbindungen bilden. Als Lösungsmittel seien beispielsweise genannt: Halogenkohlenwasserstoffe, wie Chloroform, Tetrachlorkohlenstoff, Ester, wie Essigsäure- und Benzoesäurealkylester, Lactone, wie Butyrolacton und Caprolacton, Ketone, wie Isobutyl-methylketon, Acetophenon und Cyclohexanon, Ether, wie Glykolmonomethyletheracetat, Diethylenglykolmonomethylether und Diethylenglykoldimethylether, substituierte Amide, wie Dimethylformamid und N-Methylpyrrolidon, Nitrile, Sulfoxide und Sulfone.

Besonders bevorzugt für das erfindungsgemässe Verfahren sind Lösungsmittel von geringer Polarität, wie z.B. höhere Aliphaten, Tetralin, Decalin und Alkylaromaten. Selbstverständlich können auch Gemische der einzelnen genannten Verbindungen als Lösungsmittel verwendet werden. Es ist erfindungsgemäss aber auch möglich, auf das Lösungsmittel zu verzichten und die Reaktion in der Schmelze der Reaktanten durchzuführen.

Das erfindungsgemässe Verfahren wird üblicherweise bei Normaldruck durchgeführt. Es ist aber auch möglich, das erfindungsgemässe Verfahren bei Unter- bzw. einem Überdruck durchzuführen.

Das erfindungsgemässe Verfahren kann sowohl diskontinuierlich als auch kontinuierlich durchgeführt werden.

Die Durchführung der erfindungsgemässen Reaktion erfolgt bevorzugt in der W eise, das das Säureanhydrid und der Katalysator in der Schmelze oder in einem der aufgeführten Lösungsmittel vorgelegt werden und bei Temperaturen von 100 bis 220°C, vorzugswseise von 150 bis 180°C, der Alkohol in Substanz oder in Lösung so eingetropft oder eingetragen wird, dass ein stärkerer Anstieg oder Abfall der Reaktionstemperatur, etwa durch zu starken Rückfluss bei leicht flüchtigen Alkoholen, vermieden wird. Der Reaktionsverlauf lässt sich am IR-Spektrum über die Carbonyl-Banden des zyklischen Carbonsäureanhydrids und des Halbesters verfolgen. Im allgemeinen

werden pro Mol Säureanhydrid ein Val der Hydroxyl-Verbindung eingesetzt, doch sind auch Abweichungen von diesem Mengenverhältnis zuweilen vorteilhaft.

Nach dem erfindungsgemässen Verfahren können Fumarsäuremonoester der Formel (IV)

$$R^3\text{-O-}\overset{O}{\overset{\|}{C}}\text{-CH=CH-}\overset{O}{\overset{\|}{C}}\text{-OH} \qquad (IV)$$

in der
$R^3$ die obengenannte Bedeutung hat,
hergestellt werden.

Die nach dem erfindungsgemässen Verfahren hergestellten Fumarsäureester sind Ausgangsmaterialien für die Herstellung von Kunststoffen vom Polyhydantointyp.

Ausserdem ist aus der DE-OS 2 552 634 die Verwendung von Fumarsäureester als Comonomere zur Herstellung von Lösungs- und Emulsionscopolymeren bekannt.

*Beispiel 1*

*Fumarsäuremonoethylester*

In die Lösung von 1568 g Maleinsäureanhydrid und 16 g Jod als Katalysator in 1600 g eines technischen Alkyl-aromaten-Gemisches als Lösungsmittel werden bei 160°C innerhalb von etwa 1 Stunde 736 g Ethanol eingetropft. Dann wird noch eine halbe Stunde nachgerührt. Das Reaktionsgemisch wird noch heiss filtriert und das Filtrat über eine Kolonne fraktioniert. Man erhält bei 0,4 mbar/106-108°C 1810 g Fumarsäuremonoethylester vom Schmp. 106 - 108°C.

Analyse:

| | | |
|---|---|---|
| ber.: | C 50,0 | H 5,6 % |
| gef.: | C 50,3 | H 5,8 % |

*Beispiel 2*

*Fumarsäuremonoisopropylester*

In 1750 g eines technischen Alkyl-aromaten-Gemisches als Lösungsmittel werden 882 g Maleinsäureanhydrid, 2,9 g Jod, 1 g Anilin und 1 g Hydrochinon gelöst und die Lösung auf 160°C aufgeheizt. Dann werden bei dieser Temperatur im Verlauf von 1,5 Stunden 567 g Isopropanol zugetropft und anschliessend noch eine halbe Stunde nachgerührt. Aus dem Reaktionsgemisch werden durch Filtration geringe Anteile Fumarsäure abgetrennt.

Die Destillation des Filtrats über eine Silbermantelkolonne ergibt bei 0,14 mbar/100-102°C 1110 g Fumarsäuremonoisopropylester vom Schmp. 50 bis 51°C.

Analyse:

| | | |
|---|---|---|
| ber.: | C 53,2 | H 6,3 % |
| gef.: | C 53,4 | H 6,2 % |

*Beispiel 3*

*Fumarsäuremonododecylester*

In die Lösung von 392 g Maleinsäureanhydrid und 4 g Jod in 400 g eines technischen Alkyl-aromaten-Gemisches als Lösungsmittel werden bei 160°C 744 g Dodecanol eingetropft. Das Reaktionsgemisch wird noch heiss filtriert. Aus dem Filtrat kristallisieren beim Abkühlen 820 g Fumarsäuredodecylester in farblosen Kristallen vom Schmp. 80-82°C.

Analyse:

| | | |
|---|---|---|
| ber.: | C 67,6 | H 9,9 % |
| gef.: | C 67,6 | H 9.8 % |

*Beispiel 4*

*Fumarsäuremonomethylester*

In 1000 g eines technischen Alkyl-aromaten-Gemisches als Lösungsmittel, 980 g Maleinsäureanhydrid und 10 g Jod werden bei 160°C 320 g Methanol eingetropft. Das Reaktionsgemisch wird heiss filtriert. Beim Erkalten kristallisiert der Fumarsäuremonomethylester in farblosen Kristallen aus. Die Umkristallisation aus Toluol ergibt 857 g reinen Fumarsäuremonomethylester vom Schmp. 142 bis 144°C.

Analyse:

| | | |
|---|---|---|
| ber.: | C 46,2 | H 4,6 % |
| gef.: | C 46,2 | H 4,6 % |

*Beispiel 5*

*Fumarsäuremonobenzylester*

In 200 g eines technischen Alkyl-aromaten-Gemisches als Lösungsmittel, 196 g Maleinsäureanhydrid und 2 g Jod werden bei 160°C im Verlauf von 45 Minuten 216 g Benzylalkohol eingetropft. Anschliessend wird heiss filtriert, nach dem Erkalten erneut filtriert und der Filtrierrückstand aus Acetonitril umkristallisiert. Man erhält den Fumarsäuremonobenzylester in farblosen Kristallen Schmp. 108 bis 110°C.

Analyse:

| | | |
|---|---|---|
| ber.: | C 64,1 | H 4,9 % |
| gef.: | C 64,2 | H 4,9 % |

*Beispiel 6*

*Fumarsäuremonocyclohexylester*

In die Lösung von 196 g Maleinsäureanhydrid und 2 g Jod in 200 g eines technischen Alkyl-aromaten-Gemisches als Lösungsmittel werden bei 160°C im Verlaufe von 50 Minuten 200 g Cyclohexanol eingetropft, im Wasserstrahlvakuum eingedampft und der Rückstand aus Leichtbenzin/Toluol umkristallisiert. man erhält den Fumarsäurecyclohexylester in farblosen Kristallen vom Schmp. 86-88°C.

Analyse:

| | | |
|---|---|---|
| ber.: | C 60,6 | H 7,1 % |
| gef.: | C 60,7 | H 7,1 % |

*Beispiel 7*

*Fumarsäuremonoallylester*

196 g Maleinsäureanhydrid und 2 g Jod werden in 200 g eines technischen Alkyl-aromaten-Gemisches als Lösungsmittel gelöst. Im Verlauf einer Stunde werden bei 160°C 116 g Allylalkohol zugetropft. Anschliessend wird heiss filtriert. Beim Erkalten kristallisiert der Fumarsäuremonoallylester aus, wird

abgesaugt und aus Leichtbenzin umkristallisiert. Man erhält farblose Kristalle vom Schmp. 66-67°C.

Analyse:

| | | |
|---|---|---|
| ber.: | C 53,9 | H 5,1 % |
| gef.: | C 53,9 | H 5,1 % |

*Beispiel 8*

*Butan-(1,4)-bis-fumarsäuremonoester*

In 200 g eines technischen Alkyl-aromaten-Gemisches als Lösungsmittel, 196 g Maleinsäureanhydrid, 2 g Jod und 1 g Ammoniumchlorid werden bei 160°C 90 g Butan-(1,4)-diol eingetropft. Man rührt noch 30 Minuten bei 160°C nach, saugt nach dem Erkalten ab und erhält nach Umkristallisation aus Acetonitril/Dimethylformamid den Bisester als weisse kristalline Substanz vom Schmp. 172 bis 174°C.

Analyse:

| | | |
|---|---|---|
| ber.: | C 50,4 | H 4,9 % |
| gef.: | C 50,5 | H 4,9 % |

*Beispiel 9*

*Fumarsäuremono-[1,3-dioxan-(5)-ethyl-(5)--methyl]-ester*

In die Lösung von 196 g Maleinsäureanhydrid und 2 g Jod in 200 g eines technischen Alkyl-aromaten-Gemisches als Lösungsmittel werden im Verlaufe von 2 Stunden 292 g 1,3-Dioxan-(5)-ethyl-(5)-methanol anteilweise bei 160°C eingetragen. Das Reaktionsgemisch wird bei 120°C zur Abtrennung von festen Anteilen filtriert und das Filtrat dann im Vakuum eingedampft. Nach Umkristallisation aus Toluol/Cyclohexan (1 : 1) erhält man den Monoester mit einem Schmp. von 77-78°C.

Analyse:

| | | |
|---|---|---|
| ber.: | C 54,1 | H 6,6 % |
| gef.: | C 54,0 | H 6,6 % |

*Beispiel 10*

*Fumarsäuremonopropargylester*

In 380 g eines technischen Alkyl-aromaten-Gemisches als Lösungsmittel, 192 g Maleinsäureanhydrid, 1,9 g Jod und 1 g Ammoniumchlorid werden bei 160°C 112 g Propargylalkohol eingetropft. Beim Erkalten fällt der Monoester aus und wird nach dem Absaugen aus Acetonitril und Toluol umkristallisiert. Man erhält den reinen Fumarsäuremonopropargylester mit einem Schmp. von 102-103°C.

Analyse:

| | | |
|---|---|---|
| ber.: | C 54,6 | H 3,9 % |
| gef.: | C 54,4 | H 3,9 % |

**Patentanspruch**

Verfahren zur Herstellung von Fumarsäuremonoestern, dadurch gekennzeichnet, dass man in eine Lösung oder Schmelze aus gegebenenfalls substituiertem Maleinsäureanhydrid in Gegenwart eines cis-trans-Katalysators eine Hydroxyverbindung im Temperaturbereich von 100 bis 220°C der Umsetzung entsprechend einträgt.

**Claim**

Process for the preparation of fumaric acid monoesters, characterised in that a hydroxyl compound is introduced, within a temperature range of 100 to 220°C and in accordance with the course of the reaction, into a solution or melt of optionally substituted maleic anhydride in the presence of a cis-trans catalyst.

**Revendication**

Procédé de production de monoesters d'acide fumarique, caractérisé en ce qu'on introduit un composé hydroxylique dans un intervalle de température de 100 à 220°C conformément à la réaction dans une solution ou dans une masse fondue d'anhydride d'acide maléique éventuellement substitué, en présence d'un catalyseur cis-trans.